# EUROPÄISCHE PATENTSCHRIFT

(11) **EP 0 860 424 B1**
(45) Veröffentlichungstag und Bekanntmachung des Hinweises auf die Patenterteilung: **19.12.2001**
(21) Anmeldenummer: 98101526.6
(22) Anmeldetag: 29.01.1998
(51) Int. Cl.: C07C 209/60

(54) **Verfahren zur Herstellung von Aminen aus Olefinen an NU-85 Zeolithen**
Process for the preparation of amines from olefines using NU-85 zeolites
Procédé pour la préparation d'amines à partir d'oléfines utilisant des zéolithes du type NU-85

(30) Priorität: 25.02.1997 DE 19707386
(43) Veröffentlichungstag der Anmeldung: 26.08.1998
(73) Patentinhaber: BASF AKTIENGESELLSCHAFT, 67056 Ludwigshafen (DE)
(72) Erfinder: Eller, Karsten, Dr., 67061 Ludwigshafen (DE); Kummer, Rudolf, Dr., 67227 Frankenthal (DE); Dernbach, Matthias, Dr., 69214 Eppelheim (DE); Lützel, Hans-Jürgen, 67459 Böhl-Iggelheim (DE)

(56) Entgegenhaltungen:
- EP-A- 0 462 745
- EP-A- 0 752 410
- EP-A- 0 754 676
- NORITAKA MIZUNO ET AL: "FACTORS CONTROLLING AMINATION OF 2-METHYLPROPENE OVER PROTON-EXCHANGED ZEOLITE CATALYSTS" JOURNAL OF THE CHEMICAL SOCIETY. FARADAY TRANSACTIONS, Bd. 89, Nr. 18, 21. September 1993, Seiten 3513-3514, XP000387157

## Beschreibung

Die vorliegende Erfindung betrifft ein Verfahren zur Herstellung von Aminen durch Umsetzung von Ammoniak oder primären oder sekundären Aminen mit Olefinen bei erhöhten Temperaturen und Drücken in Gegenwart von NU-85 Zeolithen.

Eine Übersicht über die Methoden zur Aminierung von Olefinen wird in "Functionalisation of Alkenes: Catalytic Amination of Monoolefins", J.J. Brunet et al. J. Mol. Catal., 49 (1989), Seiten 235 bis 259 gegeben.

Grundsätzlich gibt es zwei Katalysemechanismen. Das Olefin wird über einen Metallkomplex koordiniert. Diese aktivierte Spezies kann von dem nucleophilen Amin angegriffen werden und ein höher aminiertes Produkt bilden. Das Amin kann an Säurezentren oder an Metallzentren (via Metallamide) chemisorbiert werden und so aktiviert mit dem Olefin umgesetzt werden.

Gut geeignete Katalysatoren sind Zeolithe. Sie zeichnen sich durch eine hohe Anzahl an katalytisch aktiven Zentren aus, kombiniert mit einer großen Oberfläche. Die beschriebenen Zeolithe unterscheiden sich im Typ und in der Nachbehandlung (z.B. thermische Behandlung, Dealuminierung, Säurebehandlung, Metallioneneintausch, etc.). Beispiele hierfür finden sich in US-A-4 375 002, US-A-4 536 602, EP-A-305 564, EP-A-101 921, DE-A-42 06 992.

Aus EP-A-133 938, EP-A-431 451 und EP-A-132 736 sind Verfahren bekannt, bei denen Bor-, Gallium-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Aus CA-A-2 092 964 ist ein Verfahren zur Herstellung von Aminen aus Olefinen bekannt, bei dem BETA-Zeolithe, die als kristalline Aluminosilikate bestimmter Zusammensetzung mit einer Porengröße von mehr als 5 Å definiert sind, eingesetzt werden. Bevorzugt werden metall- oder halogenmodifizierte Beta-Zeolithe eingesetzt.

Alle Verfahren zur Synthese von Aminen aus Olefinen an diesen Katalysatoren zeichnen sich durch eine geringe Amin-Ausbeute oder geringe Raum-Zeit-Ausbeute aus, oder führen zu einer raschen Desaktivierung der Katalysatoren.

Der vorliegenden Erfindung lag daher die Aufgabe zugrunde, diesen Nachteilen abzuhelfen.

Demgemäß wurde ein neues und verbessertes Verfahren zur Herstellung von Aminen der allgemeinen Formel I 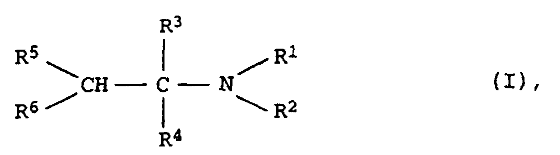 in der
- R¹,R²,R³,R⁴,R⁵,R⁶: Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl,
- R¹ und R²: gemeinsam ein C₃- bis C₉-Alkylen oder C₃- bis C₉-Alkenylen oder
- R³ oder R⁵: C₂₁- bis C₂₀₀-Alkyl, C₂₁- bis C₂₀₀-Alkenyl oder gemeinsam ein C₂- bis C₁₂-Alkylen
bedeuten, durch Umsetzung von Olefinen der allgemeinen Formel II 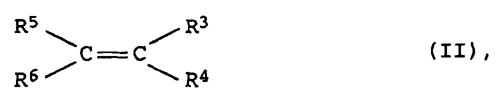 in der R³, R⁴, R⁵ und R⁶ die oben genannten Bedeutungen haben, mit Ammoniak oder primären oder sekundären Aminen der allgemeinen Formel III 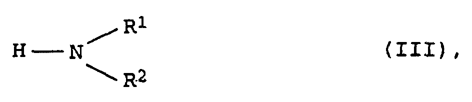 in der R¹ und R² die oben genannten Bedeutungen haben, bei Temperaturen von 200 bis 350°C und Drücken von 100 bis 300 bar in Gegenwart eines Heterogenkatalysators gefunden, welches dadurch gekennzeichnet ist, daß man als Heterogenkatalysator NU-85 Zeolithe einsetzt.

Das erfindungsgemäße Verfahren kann wie folgt durchgeführt werden:

Das Olefin II und Ammoniak oder das primäre oder sekundären Amin III kann bei Temperaturen von 200 bis 350°C, bevorzugt 220 bis 330°C, besonders bevorzugt 230 bis 320°C und Drücken von 100 bis 300 bar, bevorzugt 120 bis 300 bar, besonders bevorzugt 140 bis 290 bar in Gegenwart von NU-85 zeolithen als Katalysator z.B. in einem Druck-Reaktor umsetzt werden, und bevorzugt das erhaltene Amin abgetrennt und die nichtumgesetzten Einsatzstoffe zurückführt werden.

Das vorliegende Verfahren zeichnet sich durch eine sehr gute Ausbeute bei hoher Selektivität und bei hoher Raum-Zeit-Ausbeute aus. Zudem ist die Desaktivierung des Katalysators zurückgedrängt worden.

Das erfindungsgemäße Verfahren zeichnet sich dadurch aus, daß bereits mit niedrigem Ammoniak- bzw. Amin- Überschuß eine hohe Selektivität an gewünschtem Reaktionsprodukt erzielt und die Dimerisierung und/oder Oligomerisierung des eingesetzten Olefins vermieden wird.

Eine Ausführungsform dieses Verfahrens besteht darin, daß man Ammoniak und/oder Amine III zusammen mit dem Olefin II im molaren Verhältnis von 1:1 bis 5:1 gemischt einem Festbettreaktor zuführt und bei einem Druck von 100 bis 300 bar und einer Temperatur von 200 bis 350°C in der Gasphase oder im überkritischen Zustand umsetzt.

Aus dem Reaktionsaustrag kann das gewünschte Produkt mit Hilfe bekannter Methoden, beispielsweise Destillation oder Extraktion, erhalten und nötigenfalls mittels weiterer Trennoperationen auf die gewünschte Reinheit gebracht werden. Die nichtumgesetzten Eingangsstoffe werden in der Regel bevorzugt in den Reaktor zurückgeführt.

Man kann einfach oder mehrfach ungesättigte Olefine II, insbesondere solche mit 2 bis 10 C-Atomen bzw. deren Mischungen und Polyolefine als Ausgangsstoffe verwenden. Wegen der geringer ausgeprägten Polymerisationsneigung eignen sich Monoolefine besser als Di- und Polyolefine, doch können diese mit Hilfe höherer Ammoniak- bzw. Aminüberschüsse ebenso selektiv umgesetzt werden. Die Lage des Gleichgewichts und damit der Umsatz zum gewünschten Amin ist sehr stark vom gewählten Reaktionsdruck abhängig. Hoher Druck begünstig das Additionsprodukt, doch stellt im allgemeinen aus technischen und wirtschaftlichen Gründen der Druckbereich bis 300 bar das Optimum dar. Die Selektivität der Reaktion wird - neben Größen wie Ammoniak-/ Amin-Überschuß und Katalysator - in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Zudem ist eine Temperaturerhöhung aus thermodynamischer Sicht nicht vorteilhaft. Die Lage des Temperaturoptimums bezüglich Umsatz und Selektivität ist von der Konstitution des Olefins, des eingesetzten Amins und des Katalysators abhängig und liegt meist im Bereich von 200 bis 350°C.

Als Katalysatoren für die Aminierung von Olefinen eignen sich NU-85 Zeolithe, die aus EP-A-462 745 bekannt sind. Von der Struktur her handelt es sich um einen Mischkristall aus NU-87 und EU-1, er besitzt aber ein mehrdimensionales Kanalsystem (Shannon, Proc. 9th Int. Zeolite Conf. (1992) 389), während der EU-1 (EUO) ein eindimensionaler Zehnringzeolith, d.h. ein Pentasil ist. Alle Mischkristalle zwischen dem reinen EU-1 und dem reinen NU-87 (NES) werden als NU-85 bezeichnet.

Neben den NU-85 Zeolithen mit Aluminium als dreiwertigem Element in der SiO₂-Matrix, wie sie z.B. in EP-A-462 745 beschrieben sind, sind im Sinne dieser Anmeldung weiterhin andere Elemente möglich, wenn durch ihren Einbau acide Zentren geschaffen werden. Dies ist beispielsweise bei Borzeolithen, Eisenzeolithen oder Galliumzeolithen der Fall. Das molare Verhältnis von SiO₂ zu den Oxiden der dreiwertigen Elemente, das sogenannte Modul SiO₂/M₂O₃ (M = Al, B, Ga, Fe), kann je nach Zeolithklasse zwischen nahezu unendlich bis zu einigen Zehn variieren. Durch die Variation des Moduls kann die Aktivität der Zeolithe auf die jeweilige Aminierungsreaktion maßgeschneidert werden, um optimale Ergebnisse zu erhalten. Anstelle des dreiwertigen Elements kann auch Silizium isomorph durch andere vierwertige Elemente substituiert werden, so zum Beispiel durch Ge, Ti oder Sn.

Neben den klassischen Zeolithen auf SiO₂-Basis können analoge Strukturen auch auf Basis von Aluminiumphosphaten realisiert werden, den sogenannten AlPOs. Enthalten diese Aluminium und Phosphor im Verhältnis größer 1, so sind sie ebenfalls sauer und können im Sinne der Erfindung eingesetzt werden. Ist ein Teil des Phosphors und/oder gleichzeitig Aluminium und Phosphor durch Silizium ersetzt, so erhält man die sogenannten SAPOs, die ebenfalls sauer sind. Kommen neben Aluminium und Phosphor noch verschiedene Metallionen wie beispielsweise Li, B, Be, Mg, Ti, Mn, Fe, Co, Zn, Ga, Ge, As vor, so spricht man von MeAPOs, oder bei gleichzeitiger Anwesenheit von Silizium von MeAPSOs, bei denen die negative Ladung des MeₐAl_{b}P_{c}Si_{d}Oₑ-Gerüstes jeweils durch Kationen kompensiert wird. Alle derartigen Molekularsiebe mit NU-85 Struktur fallen unter die erfindungsgemäßen Katalysatoren.

Die erfindungsgemäßen NU-85 Zeolithe können als solche verformt werden, oder aber auch mit einem Bindemittel im Verhältnis 98:2 bis 40:60 Gew.-% zu Strängen oder Tabletten. Als Bindemittel eignen sich verschiedene Aluminiumoxide, bevorzugt Boehmit, amorphe Aluminosilikate mit einem SiO₂/Al₂O₃-Verhältnis von 25:75 bis 95:5, Siliciumdioxid, bevorzugt hochdisperses SiO₂, Gemische aus hochdispersem SiO₂ und hochdispersem Al₂O₃, hochdisperses TiO₂ sowie Tone. Nach der Verformung werden die Extrudate oder Preßlinge zweckmäßig bei 110°C/16 Stunden getrocknet und bei 200 bis 500°C/2 bis 16 Stunden calciniert, wobei die Calcinierung auch direkt im Aminierungsreaktor erfolgen kann.

Zur Erhöhung der Selektivität, der Standzeit und der Anzahl der möglichen Regenerierungen kann man verschiedene Modifizierungen an den erfindungsgemäßen NU-85 Zeolithkatalysatoren vornehmen.

Eine Modifizierung der Katalysatoren besteht darin, daß man die unverformten oder die verformten Zeolithe mit Alkalimetallen wie Na und K, Erdalkalimetallen wie Ca, Mg, Erdmetallen wie T1, Übergangsmetallen wie z.B. Ti, Zr, Mn, Fe, Mo, Cu, Zn, Cr, Edelmetallen und/oder seltenen Erdmetallen wie z.B. La, Ce oder Y ionenaustauschen bzw. dotieren kann.

Eine vorteilhafte Ausführungsform besteht darin, daß man die verformten erfindungsgemäßen NU-85 Zeolithe in einem Strömungsrohr vorlegt und bei 20 bis 100°C z.B. ein Halogenid, ein Acetat, ein Oxalat, ein Citrat oder ein Nitrat der oben beschriebenen Metalle in gelöster Form darüberleitet. Ein derartiger Ionenaustausch kann z.B. an der Wasserstoff-, Ammonium- und Alkaliform der erfindungsgemäßen NU-85 Zeolithe vorgenommen werden.

Eine weitere Möglichkeit der Metallaufbringung auf die erfindungsgemäßen NU-85 Zeolithe besteht darin, daß man das Material z.B. mit einem Halogenid, einem Acetat, einem Oxalat, einem Citrat, einem Nitrat oder einem Oxid der oben beschriebenen Metalle in wäßriger oder alkoholischer Lösung imprägniert.

Sowohl an einen Ionenaustausch als auch an eine Imprägnierung kann man eine Trocknung, wahlweise eine abermalige Calcination anschließen. Bei metalldotierten NU-85 Zeolithen kann eine Nachbehandlung mit Wasserstoff und/oder mit Wasserdampf günstig sein.

Eine weitere Möglichkeit der Modifizierung besteht darin, daß man die erfindungsgemäßen NU-85 Zeolithe - verformt oder unverformt - einer Behandlung mit Säuren, wie Salzsäure (HCl), Flußsäure (HF), Schwefelsäure (H₂SO₄), Phosphorsäure (H₃PO₄), Oxalsäure (HO₂C-CO₂H) oder deren Gemischen unterwirft.

Eine besondere Ausführungsform besteht darin, daß man die erfindungsgemäßen NU-85 Zeolithe vor der Verformung mit einer der genannten Säuren 0,001 n bis 2 n, bevorzugt 0,05 bis 0,5 n 1 bis 100 Stunden unter Rückfluß behandelt. Nach Abfiltrieren und Auswaschen wird in der Regel bei 100 bis 160°C getrocknet und bei 200 bis 600°C calciniert. Eine weitere besondere Ausführungsform liegt in einer Säure-Behandlung der erfindungsgemäßen NU-85 Zeolithe nach ihrer Verformung mit Bindemittel. Hierbei wird der erfindungsgemäße Zeolith in der Regel 1 bis 3 Stunden zwischen 60 und 80°C mit einer 3 bis 25%igen, insbesondere mit einer 12 bis 20%igen Säure behandelt, anschließend ausgewaschen, bei 100 bis 160°C getrocknet und bei 200 bis 600°C calciniert. Auch hier kann die Calcinierung wieder direkt im Aminierungsreaktor erfolgen.

Eine andere Möglichkeit der Modifizierung ist gegeben durch einen Austausch mit Ammonsalzen, z.B. mit NH₄Cl oder mit Mono-, Di- oder Polyaminen. Hierbei wird der mit Bindemittel verformte Zeolith in der Regel bei 60 bis 80°C mit 10 bis 25%iger, bevorzugt 20%iger NH₄Cl-Lösung 2 h kontinuierlich in gewichtsmäßiger Zeolith/Ammonchlorid-Lösung von 1:15 ausgetauscht und danach bei 100 bis 120°C getrocknet.

Eine weitere Modifikation, die an den erfindungsgemäßen Zeolithen vorgenommen werden kann, ist die Dealuminierung im Falle von Aluminium-Zeolithen, bei der ein Teil der Aluminiumatome durch Silicium ersetzt wird oder die Zeolithe durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503. Bei anderen dreiwertigen Oxiden kann entsprechend das Modul vergrößert werden, indem ein Teil des Bors, des Eisens oder des Galliums herausgelöst oder durch Silicium ersetzt wird.

Die Katalysatoren kann man als Stränge mit Durchmessern von z.B. 1 bis 4 mm, als Kugeln oder als Tabletten mit z.B. 3 bis 5 mm Durchmesser für die Aminierung der Olefine einsetzen. Andere Formkörper sind ebenfalls möglich.

Aus dem beispielsweise zu Strängen verformten Katalysator kann man durch Mahlen und Sieben ein Wirbelgut in der Größe von 0,1 bis 0,8 mm erhalten.

Die Substituenten R¹, R², R³, R⁴, R⁵ und R⁶ in den Verbindungen I, II und III haben folgende Bedeutungen:
R¹,R²,R³,R⁴,R⁵,R⁶
   - Wasserstoff,
   - C₁- bis C₂₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
   - C₂- bis C₂₀-Alkenyl, bevorzugt C₂- bis C₁₂-Alkenyl, besonders bevorzugt C₂- bis C₈-Alkenyl wie Vinyl und Allyl,
   - C₂- bis C₂₀-Alkinyl, bevorzugt C₂- bis C₈-Alkinyl, insbesondere C₂H und Propargyl,
   - C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
   - C₄- bis C₂₀-Alkyl-cycloalkyl, bevorzugt C₄- bis C₁₂-Alkylcycloalkyl, besonders bevorzugt C₅- bis C₁₀-Alkyl-cycloalkyl,
   - C₄- bis C₂₀-Cycloalkyl-alkyl, bevorzugt C₄- bis C₁₂-Cycloalkyl-alkyl, besonders bevorzugt C₅- bis C₁₀-Cycloalkylalkyl,
   - Aryl wie Phenyl, 1-Naphthyl und 2-Naphthyl, bevorzugt Phenyl,
   - C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
   - C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl, 2-Phenylethyl,
R¹ und R²
   - gemeinsam ein C₃- bis C₉-Alkylen oder C₃- bis C₉-Alkenylen, bevorzugt -(CH₂)₄-, -(CH₂)₅-, -(CH₂)₇- und -CH=CH-CH=CH-,
R³ oder R⁵
   - C₂₁- bis C₂₀₀-Alkyl, bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
   - C₂₁- bis C₂₀₀-Alkenyl, bevorzugt C₄₀- bis C₂₀₀-Alkenyl, besonders bevorzugt C₇₀- bis C₁₇₀-Alkenyl,
R³ und R⁵
   - gemeinsam ein C₂- bis C₁₂-Alkylen, bevorzugt ein C₃- bis C₈-Alkylen, besonders bevorzugt -(CH₂)₃-, -(CH₂)₄-, -(CH₂)₅-, -(CH₂)₆- und -(CH₂)₇-, insbesondere -(CH₂)₃- und -(CH₂)₄-.

### Beispiele

### Katalysatorsynthesen

### Katalysator A: Na-NU-85

Lösung A wurde aus 13.3 g NaOH und 6.6 g Natriumaluminat in 379 g Wasser angesetzt. Lösung B wurde aus 78 g Hexamethoniumbromid (BrMe₃N-(CH₂)₆-NMe₃Br) in 378 g Wasser angesetzt. Lösung C wurde aus 77 g Aerosil® 50 in 396 g Wasser angesetzt. Lösung A wurde aus 77 g Aerosil® 50 in 396 g Wasser angesetzt. Lösung A wurde mit Lösung B gemischt und unter Rühren zu Lösung C gegeben. Es wurde noch weiter gerührt, bis ein homogenes Gel entstand. Die Mischung wurde dann in einen Autoklaven überführt und 260 h bei 160°C unter dem Eigendruck gerührt kristallisiert. Der gebildete Zeolith wurde abfiltriert und gewaschen, 16 h bei 110°C getrocknet und 16 h bei 500°C kalziniert. Nach Röntgenpulverdiffraktometrie handelte es sich um NU-85.

### Katalysator B: H-NU-85

50 g Katalysator A wurden mit 750 g einer 20 %igen NH₄Cl-Lösung bei 80°C für 2 h gerührt und anschließend abfiltriert und mit 1 Liter Wasser gewaschen. Nach erneutem NH₄Cl-Austausch und Nachwaschen wurde der Zeolith 2 h bei 120°C getrocknet und 5 h bei 500°C kalziniert. Der gesamte Vorgang wurde dann noch einmal wiederholt. Das ausgetauschte Pulver besaß einen Rest-Natrium-Gehalt von unter 0.005 %.

42 g des ausgetauschten Zeolithen wurden mit 28 g Boehmit und 1.4 g Ameisensäure im Kneter kompaktiert und unter Wasserzusatz (53 ml) 50 min verknetet. in einer Strangpresse wurden mit einem Pressdruck von 70 bar 2 mm Stränge erzeugt und diese 4 h bei 120°C getrocknet und 16 h bei 500°C kalziniert.

### Katalysator C: Na-NU-85

### Katalysator C wurde analog zu Katalysator A synthetisiert.

### Katalysator D: H-NU-85

Katalysator D wurde analog zu Katalysator B ionenausgetauscht, aber aus Katalysator C. 40 g des ausgetauschten Zeolithen wurden mit 27 g Boehmit und 1.5 g Ameisensäure im Kneter kompaktiert und unter Wasserzusatz (61 ml) 40 min verknetet. In einer Strangpresse wurden mit einem Pressdruck von 40 bar 2 mm Stränge erzeugt und diese 4 h bei 120°C getrocknet und 16 h bei 500°C kalziniert. Der Rest-Natrium-Gehalt lag unter 0.01 %.

### Katalysator E: H-NU-85

25 g Katalysator D wurden in ein beheizbares Glasrohr eingefüllt und 750 g einer 20 %igen NH₄Cl-Lösung bei 80°C für 2 h im Kreislauf darübergepumpt. Anschließend wurden 6 l H₂O im geraden Durchgang übergeleitet und der NH₄Cl-Austausch noch einmal wiederholt. Nach Waschen mit 10 l Wasser wurde der Zeolith 4 h bei 120°C getrocknet und 5 h bei 500°C kalziniert.

### Aminierungsbeispiele

### Aminierung von Isobuten

Die Versuche wurden in einem Rohrreaktor (6 mm Innendurchmesser) unter isothermen Bedingungen bei 260 bis 300°C und einem Druck von 280 bar mit einem Gemisch aus Isobuten und Ammoniak im molaren Verhältnis 1:1,5 durchgeführt. Die Reaktionsprodukte wurden im Gaschromatographen analysiert. Die Ergebnisse mit den verschiedenen Katalysatoren sind in Tabelle 1 zusammengestellt.

**Tabelle 1:**

| Katalysator | Temperatur [°C] | tert.-Butylamin-Ausbeute [Gew.-%] | | | Litergewicht [kg/l] |
|---|---|---|---|---|---|
| | | WHSV 0,75 [g/g·h] | WHSV 1,5 [g/g·h] | WHSV 3 [g/g·h] | |
| D | 260 | 22,47 | | | 0,48 |
| D | 270 | 23,36 | 19,42 | 15,40 | 0,48 |
| D | 280 | 20,92 | | 17,78 | 0,48 |
| E | 260 | 16,94 | | | 0,48 |
| E | 270 | 19,03 | 16,43 | 11,77 | 0,48 |
| E | 280 | 19,58 | 17,99 | 14,32 | 0,48 |
| E | 300 | | | 13,30 | 0,48 |

### Aminierung von Cyclohexen

In einen 0.3 l Rührautoklaven wurden jeweils 10 g Katalysator B sowie Cyclohexen gegeben. Nach dem Verschließen wurde Ammoniak (30 ml) und 100 bar Stickstoff aufgepreßt und nach dem Aufheizen auf Reaktionstemperatur 16 h gerührt. Die Ergebnisse sind in Tabelle 2 angegeben, wobei sich der Umsatz auf Cyclohexen und die Selektivität auf Cyclohexylamin bezieht:

| NH₃/C₆H₁₀ [mol/mol] | Temperatur [°C] | Druck [bar] | Umsatz [%] | Selektivität [%] |
|---|---|---|---|---|
| 1,5:1 | 300 | 275 | 4 | 61 |
| 3:1 | 300 | 400 | 5 | 77 |
| 1,5:1 | 280 | 344 | 1 | 67 |

## Patentansprüche

1. Verfahren zur Herstellung von Aminen der allgemeinen Formel I 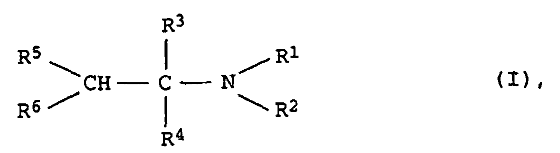 in der
R¹,R²,R³,R⁴,R⁵,R⁶ Wasserstoff, C₁- bis C₂₀-Alkyl, C₂- bis C₂₀-Alkenyl, C₂- bis C₂₀-Alkinyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl oder C₇- bis C₂₀-Aralkyl oder
R¹ und R² gemeinsam ein C₃- bis C₉-Alkylen oder C₃- bis C₉-Alkenylen oder
R³ oder R⁵ C₂₁- bis C₂₀₀-Alkyl, C₂₁- bis C₂₀₀-Alkenyl oder gemeinsam ein C₂- bis C₁₂-Alkylen
bedeuten, durch Umsetzung von Olefinen der allgemeinen Formel II 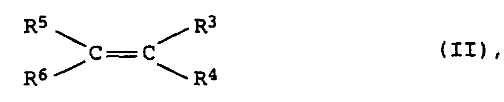 in der R³, R⁴, R⁵ und R⁶ die oben genannten Bedeutungen haben, mit Ammoniak oder primären oder sekundären Aminen der allgemeinen Formel III 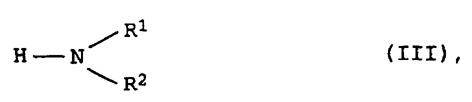 in der R¹ und R² die oben genannten Bedeutungen haben, bei Temperaturen von 200 bis 350°C und Drücken von 100 bis 300 bar in Gegenwart eines Heterogenkatalysators, **dadurch gekennzeichnet, daß** man als Heterogenkatalysator NU-85 Zeolithe einsetzt.

2. Verfahren zur Herstellung von Aminen I nach Anspruch 1, **dadurch gekennzeichnet, daß** man das gebildete Amin I abtrennt und die nichtumgesetzten Einsatzstoffe II und III zurückführt.

3. Verfahren zur Herstellung von Aminen nach den Ansprüchen 1 bis 2, **dadurch gekennzeichnet, daß** man als Olefin II Isobuten, Diisobuten, Cyclopenten, Cyclohexen oder Polyisobuten einsetzt.

4. Verfahren zur Herstellung von Aminen nach den Ansprüchen 1 bis 3, **dadurch gekennzeichnet, daß** man als Heterogenkatalysatoren NU-85 Zeolithe in der H-Form einsetzt.

5. Verfahren zur Herstellung von Aminen nach den Ansprüchen 1 bis 4, **dadurch gekennzeichnet, daß** man als Heterogenkatalysatoren NU-85 Zeolithe, die mit einer Säure, insbesondere einer aus der Gruppe Salzsäure, Flußsäure, Schwefelsäure, Phosphorsäure, Oxalsäure oder deren Gemischen, behandelt sind, einsetzt.

6. Verfahren zur Herstellung von Aminen nach den Ansprüchen 1 bis 5, **dadurch gekennzeichnet, daß** man als Heterogenkatalysatoren NU-85 Zeolithe, die mit einem oder mehreren Übergangsmetallen dotiert sind, einsetzt.

7. Verfahren zur Herstellung von Aminen nach den Ansprüchen 1. bis 6, **dadurch gekennzeichnet, daß** man als Heterogenkatalysatoren NU-85 Zeolithe, die mit einem oder mehreren Elementen der Seltenen Erden dotiert sind, einsetzt.

8. Verfahren zur Herstellung von Aminen nach den Ansprüchen 1 bis 7, **dadurch gekennzeichnet, daß** man als Heterogenkatalysatoren NU-85 Zeolithe in der Ammoniumform einsetzt.

9. Verfahren zur Herstellung von Aminen nach den Ansprüchen 1 bis 8, **dadurch gekennzeichnet, daß** man als Heterogenkatalysatoren NU-85 Zeolithe einsetzt, die mit einem oder mehreren Elementen aus der Gruppe der Alkali-, Erdalkalimetalle oder Erdmetalle dotiert sind.

10. Verfahren zur Herstellung von Aminen nach den Ansprüchen 1 bis 9, **dadurch gekennzeichnet, daß** man als Heterogenkatalysatoren NU-85 Zeolithe einsetzt, die mit einem Bindemittel verformt und bei Temperaturen von 200 bis 600°C calciniert sind.

11. Verfahren zur Herstellung von Aminen nach den Ansprüchen 1 bis 10, **dadurch gekennzeichnet, daß** man als Heterogenkatalysatoren dealuminierte oder deborierte NU-85 Zeolithe einsetzt.

## Claims

1. A process for the preparation of an amine of the formula I 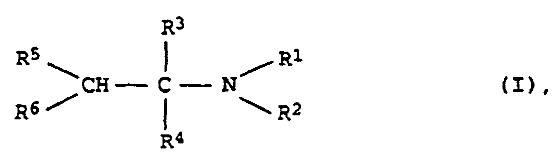 where
R¹,R²,R³,R⁴,R⁵ and R⁶ are each hydrogen, C₁-C₂₀-alkyl, C₂-C₂₀-alkenyl, C₂-C₂₀-alkynyl, C₃-C₂₀-cycloalkyl, C₄-C₂₀-alkylcycloalkyl, C₄-C₂₀-cycloalkylalkyl, aryl, C₇-C₂₀-alkylaryl or C₇-C₂₀-aralkyl, or
R¹ and R² together are C₃-C₉-alkylene or C₃-C₉-alkenylene, or
R³ or R⁵ is C₂₁-C₂₀₀-alkyl or C₂₁-C₂₀₀-alkenyl or R³ and R⁵ together are C₂-C₁₂-alkylene,
by reacting an olefin of the formula II 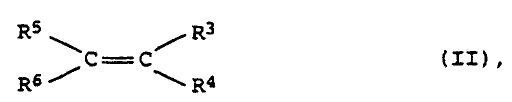 where R³, R⁴, R⁵ and R⁶ have the abovementioned meanings, with ammonia or a primary or secondary amine of the formula III 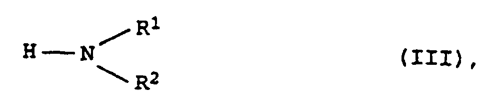 where R¹ and R² have the abovementioned meanings, at from 200 to 350°C and from 100 to 300 bar in the presence of a heterogeneous catalyst, wherein the heterogeneous catalyst used is an NU-85 zeolite.

2. A process for the preparation of an amine I as claimed in claim 1, wherein the amine I formed is isolated and the unconverted starting materials II and III are recycled.

3. A process for the preparation of an amine as claimed in claims 1 and 2, wherein the olefin II used is isobutene, diisobutene, cyclopentene, cyclohexene or polyisobutene.

4. A process for the preparation of an amine as claimed in any of claims 1 to 3, wherein the heterogeneous catalyst used is an NU-85 zeolite in the H form.

5. A process for the preparation of an amine as claimed in any of claims 1 to 4, wherein the heterogeneous catalyst used is an NU-85 zeolite which has been treated with an acid, in particular one selected from the group consisting of hydrochloric acid, hydrofluoric acid, sulfuric acid, phosphoric acid, oxalic acid and mixtures thereof.

6. A process for the preparation of an amine as claimed in any of claims 1 to 5, wherein the heterogeneous catalyst used is an NU-85 zeolite which has been doped with one or more transition metals.

7. A process for the preparation of an amine as claimed in any of claims 1 to 6, wherein the heterogeneous catalyst used is an NU-85 zeolite which has been doped with one or more rare earth elements.

8. A process for the preparation of an amine as claimed in any of claims 1 to 7, wherein the heterogeneous catalyst used is an NU-85 zeolite in the ammonium form.

9. A process for the preparation of an amine as claimed in any of claims 1 to 8, wherein the heterogeneous catalyst used is an NU-85 zeolite which has been doped with one or more elements selected from the group consisting of the alkali metals, alkaline earth metals or earth metals.

10. A process for the preparation of an amine as claimed in any of claims 1 to 9, wherein the heterogeneous catalyst used is an NU-85 zeolite which has been molded with a binder and calcined at from 200 to 600°C.

11. A process for the preparation of an amine as claimed in any of claims 1 to 10, wherein the heterogeneous catalyst used is a dealuminated or deborated NU-85 zeolite.

## Revendications

1. Procédé pour la préparation d'amines répondant à la formule générale I 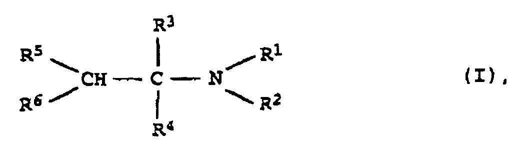 dans laquelle
R¹, R², R³, R⁴, R⁵, R⁶ représentent un atome d'hydrogène, un groupe alkyle en C₁-C₂₀, un groupe alcényle en C₂-C₂₀, un groupe alcynyle en C₂-C₂₀, un groupe cycloalkyle en C₃-C₂₀, un groupe alkyl(en C₄-C₂₀)cycloalkyle, un groupe cycloalkyl(en C₄-C₂₀)alkyle, un groupe aryle, un groupe alkyl(en C₇-C₂₀)aryle ou un groupe aralkyle en C₇-C₂₀, ou
R¹ et R² représentent ensemble un groupe alkylène en C₃-C₉ ou un groupe alcénylène en C₃-C₉ ou
R³ ou R⁵ représente un groupe alkyle en C₂₁-C₂₀₀, un groupe alcényle en C₂₁-C₂₀₀ ou représentent ensemble un groupe alkylène en C₂-C₁₂,
par mise en réaction d'oléfines répondant à la formule générale II 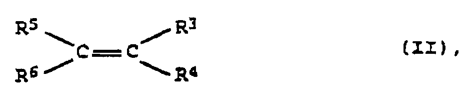 dans laquelle R³, R⁴, R⁵ et R⁶ ont les significations indiquées ci-dessus, avec de l'ammoniac ou avec des amines primaires ou secondaires répondant à la formule générale III 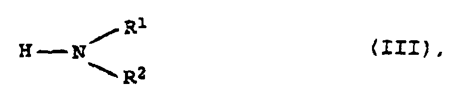 dans laquelle R¹ et R² ont les significations indiquées ci-dessus, à des températures de 200 à 350°C et sous des pressions de 100 à 300 bar en présence d'un catalyseur hétérogène, **caractérisé en ce qu'**on utilise, à titre de catalyseur hétérogène des zéolithes NU-85.

2. Procédé pour la préparation d'amines I selon la revendication 1, **caractérisé en ce qu'**on sépare l'amine I obtenue et on recycle les substances de mise en oeuvre II et III n'ayant pas réagi.

3. Procédé pour la préparation d'amines selon les revendications 1 à 2, **caractérisé en ce qu'**on met en oeuvre, à titre d'oléfine II, l'isobutène, le diisobutène, le cyclopentène, le cyclohexène ou le polyisobutène.

4. Procédé pour la préparation d'amines selon les revendications 1 à 3, **caractérisé en ce qu'**on met en oeuvre, à titre de catalyseurs hétérogènes, des zéolithes NU-85 dans la forme H.

5. Procédé pour la préparation d'amines selon les revendications 1 à 4, **caractérisé en ce qu'**on met en oeuvre, à titre de catalyseurs hétérogènes, des zéolithes NU-85 qui ont été traitées avec un acide, en particulier un acide choisi parmi le groupe constitué de l'acide chlorhydrique, de l'acide fluorhydrique, de l'acide sulfurique, de l'acide phosphorique, de l'acide oxalique ou de leurs mélanges.

6. Procédé pour la préparation d'amines selon les revendications 1 à 5, **caractérisé en ce qu'**on met en oeuvre, à titre de catalyseurs hétérogènes, des zéolithes NU-85 qui ont été dopées avec un ou plusieurs métaux transitoires.

7. Procédé pour la préparation d'amines selon les revendications 1 à 6, **caractérisé en ce qu'**on met en oeuvre, à titre de catalyseurs hétérogènes, des zéolithes NU-85 qui ont été dopées avec un ou plusieurs éléments des terres rares.

8. Procédé pour la préparation d'amines selon les revendications 1 à 7, **caractérisé en ce qu'**on met en oeuvre, à titre de catalyseurs hétérogènes, des zéolithes NU-85 dans la forme ammonium.

9. Procédé pour la préparation d'amines selon les revendications 1 à 8, **caractérisé en ce qu'**on met en oeuvre, à titre de catalyseurs hétérogènes, des zéolithes NU-85 qui ont été dopées avec un ou plusieurs éléments choisis parmi le groupe constitué de métaux alcalins, de métaux alcalino-terreux ou de métaux terreux.

10. Procédé pour la préparation d'amines selon les revendications 1 à 9, **caractérisé en ce qu'**on met en oeuvre, à titre de catalyseurs hétérogènes, des zéolithes NU-85 qui ont été façonnées avec un liant et qui ont été calcinées à des températures de 200 à 600 °C.

11. Procédé pour la préparation d'amines selon les revendications 1 à 10, **caractérisé en ce qu'**on met en oeuvre, à titre de catalyseurs hétérogènes, des zéolithes NU-85 dont on a supprimé la teneur en aluminium ou dont on a supprimé la teneur en bore.
